# EUROPEAN PATENT APPLICATION

(11) **EP 1 797 942 A1**
(43) Date of publication of application: **20.06.2007**
(21) Application number: 05077897.6
(22) Date of filing: 19.12.2005
(51) Int. Cl.: B01D 53/04, A61M 16/00

(54) **Process and device for the reuse of fluranes**

(71) Applicant: Technische Universiteit Delft, 2628 BL Delft (NL)
(72) Inventor: Davila Gomez, Ytalo Orlando, 2612 HD Delft (NL); Dijk, Jan, 6814 KJ Arnhem (NL); Mul, Guido, 2632 AA Nootdorp (NL); Kapteijn, Frederik, 1447 TK Purmerend (NL)
(74) Representative: Kupecz, Arpad

(57) **Abstract**

The invention relates to a process for the treatment of an exhaled anaesthetic gas comprising at least one anaesthetic agent, said process comprising the steps of:
- in a first step contacting said anaesthetic gas with an adsorbent to adsorb said anaesthetic agent onto said adsorbent,
- in a second step desorbing said anaesthetic agent from said adsorbent, and collecting said desorbed anaesthetic agent in a form to reuse the same.

## Description

The present invention relates to a process for the treatment of exhaled anaesthetic gas, comprising the reuse of an anaesthetic agent from an exhaled anaesthetic gas.

A comparable process is known from published US Patent application US 2003/0185735 to Hotta et al. Here a process and apparatus are described for treating a waste anaesthetic gas containing a volatile anaesthetic and nitrous oxide discharged from an operating room, comprising steps of contacting said gas with an adsorbent, thereby adsorption-removing the volatile anaesthetics contained in the waste anaesthetic gas, and then contacting the effluent gas containing nitrous oxide with a catalyst for decomposing the nitrous oxide, thereby decomposing the nitrous oxide.

A disadvantage of the process known from the prior art is that used fluranes, after desorption from the adsorbent, are transferred to a liquid phase for disposal or regeneration. This requires a decompression, a cooling and a recovery step. Hence, the process known from the state of the art involves many procedures and operations. The anaesthetic volatiles are then disposed as waste. Therefore, no reuse of the fluranes is considered in that invention, and fresh anaesthetic agents have to be added for each surgical procedure.

The present invention aims at providing a process that overcomes at least one of the problems mentioned above.

The aim is achieved by a process for treatment of exhaled anaesthetic gas, comprising the steps as indicated in claim 1. No condensation step is required for the recovery of the anaesthetic agent.

In the present invention the anaesthetic agent is administered to a patient. The patient will normally be a mammal, and is preferably a human. However, other species may be treated as well, such as birds, reptiles, et cetera. In the present application, the term "patient" encompasses all these species.

Before step 1, the exhaled gas is conducted to the adsorbent. The anaesthetic gas contains the anaesthetic agent, exhaled by a patient. The anaesthetic agent binds to the adsorbent. The adsorbent is designed to bind the anaesthetic agent to its surface. Binding can also occur inside the adsorbing material. Preferably, adsorption takes place at ambient temperature and pressure. After desorption of the anaesthetic agent, the desorbed anaesthetic agent is collected. This means that it is not evaporated into the atmosphere. After desorption, the desorbed anaesthetic agent may be directly used for readministering to the anaesthesised patient.

The desorbed anaesthetic agent does not undergo an additional cooling, decompression or recovery process. After desorption, the anaesthetic agent is supplied to the anaesthesised patient via a stream of gas,usually consisting of a mixture of O₂ and N₂O, but other gases such as air, an N₂/O₂ mixture or an anaesthetic gas/O₂ mixture is also possible. The desorbed anaesthetic agent can also be used for another patient. In this way more efficient use is made of the anaesthetic agent during the process of anaesthesia, restricting the net consumption of anaesthetic agent to the operational losses (approximately 1% of the amount actually used), and saving recovery costs.

According to another embodiment, the desorbed agent may be collected and delivered to the same or another patient at a later time. The desorbed agent can also be collected in a container such as a bottle.

Currently, the commonly used anaesthetic gas is nitrous oxide (N₂O) . N₂O forms a risk for people working in the operating room as well as for the environment. It may cause spontaneous abortion and foetal malformations. N₂O is known as a compound contributing to the greenhouse effect. As a consequence, the use of N₂O as an anaesthetic gas should be restricted and may even become legally prohibited in the future. Because of its hazardous nature, N₂O should be decomposed after use in anaesthesia, e.g. by catalytic decomposition into N₂ and O₂. The gas is decomposed at elevated temperature. Catalytic decomposition of N₂O requires the absence of anaesthetic agents such as fluranes. The present invention providing the adsorption of the anaesthetic agent is perfectly suited to be combined with a process of catalytic decomposition of N₂O.

It should be noted that the process of the present invention can also be applied to an anaesthetic gas mixture without N₂O. Gases different from N₂O may also be present in the anaesthetic gas mixture, however these gases should not be able to bind to the adsorbent used to adsorb the anaesthetic agent.

Typically used anaesthetic agents belong to the family of fluranes. Flurane anaesthetics are difficult and expensive to produce and exposure to exhaled or otherwise evaporated fluranes in the operating room is dangerous. Since the use of N₂O may become forbidden by law in the future, the demand for fluranes is expected to grow (a higher concentration of fluranes is required when they are supplied in a mixture that does not contain N₂O). Reuse of anaesthetic agents, in particular fluranes, therefore becomes an issue of increasing importance. Using the process of the present invention, all fluranes are adsorbed before the catalytic decomposition of N₂O. Furthermore, no destruction of adsorbed fluranes is required, nor are any logistics required for this. Since there is no net consumption of fluranes, there is a saving of costs, limited logistics and limited purchase. The process of the present invention provides a reliable method.

The adsorbent used to adsorb the anaesthetic agent is an adsorbent able to bind an anaesthetic agent used. When fluranes are used as the anaesthetic agent, the adsorbent may be a zeolite or a carbon or carbonaceous material or the like, as known to a man skilled in the art. An advantage of using a zeolite adsorbent is that zeolite is an incombustible material. In a preferred embodiment, the adsorbent comprises structured carbon, preferably at least partly consisting of activated carbon. Activated carbon is known for its excellent binding capacity for fluranes. "Structured" refers to the situation that the carbon (particles) have to be arranged in such a way that gas (with the fluranes) can pass easily, and that the carbon has a large adsorption surface. To remove the adsorbed anaesthetic agent, a suitable desorption process is used. In a preferred embodiment, the desorption process is effected by heating the adsorbent. The desorption temperature lies between ambient temperature, preferably the evaporation temperature of the adsorbed agent and the temperature of decomposition of the adsorbed agent, preferrably maximally 250°C. According to WO 2002 26355-A it is known to adsorb fluranes and subsequently desorb them by means of pressure reduction. However, such a pressure swing adsorption system requires multiple devices involving associated high costs. The heat-controlled system according to the present invention, moreover, can be controlled more easily than a pressure-swing adsorption system.

Heating the adsorbent preferably occurs by circulating an electric current through the structured carbon adsorbent, in this way providing heating by electric resistance. In an other embodiment, electric heating is provided by heating wires. By adjusting the electrically generated heat, the composition of the desorbed fluranes in the anaesthetic mixture administered to the patient can be controlled. Generally, the concentrations of the fluranes lie between 0 and 5 % by volume in the gas inhaled by the patient. In this way a new and reliable process is provided for reusing an anaesthetic agent in a controllable manner, without the need of an intermediate condensation step. In this part of the process the gases are not dried.

Another embodiment provides desorption by heating the flow of anaesthetic agent/O₂ that is administered to the patient, before it contacts the adsorbent. Due to the heat of the gas flow the fluranes become desorbed and are either administered to the patient, recycled, or otherwise used. (After desorption the gas flow is cooled down to a temperature at which a gas is suitable to be administered to a patient and/or is normally used in practice).

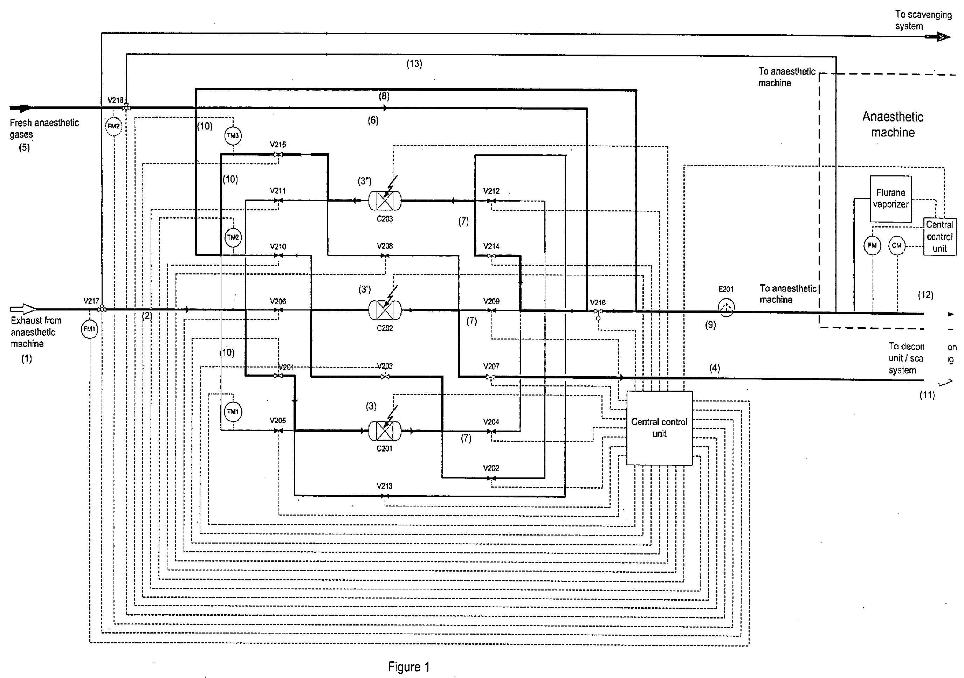

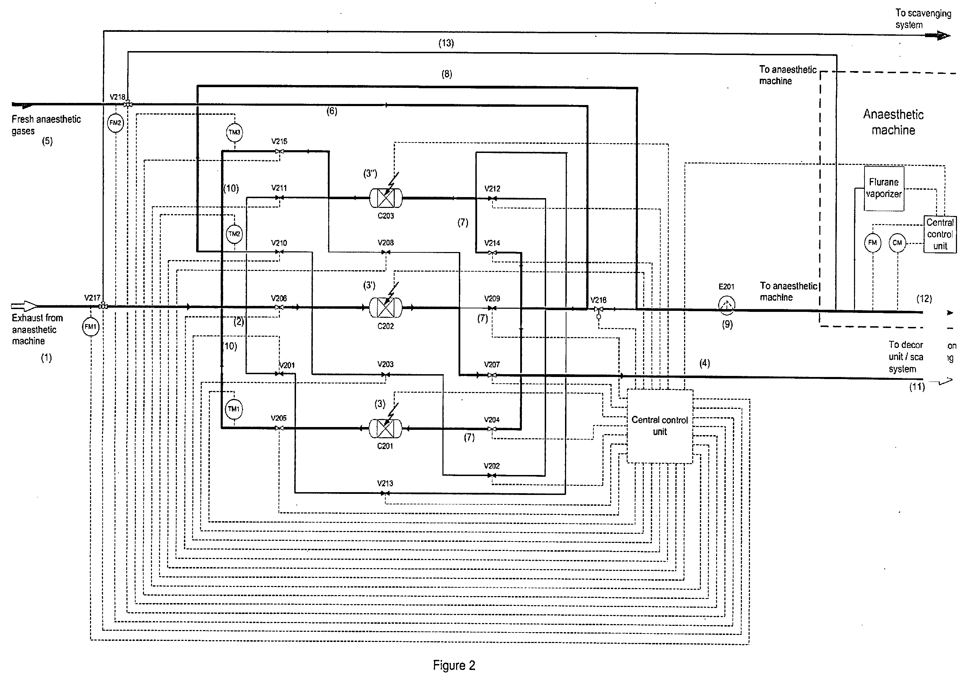

In a preferred embodiment of the process, an analyser is present in the anaesthetic gas/O₂ mixture administered to the patient, indicated with CM (concentration measuring device) in figures 1 and 2. The analyser measures at least the concentration of the anaesthetic agent in the gas to be inhaled by the patient. The analyser may for example be a flame ionisation detector or preferably an UV/VIS-cell. This kind of analysers is already present in anaesthetic machines used in the state of the art. A controller uses the signal from this analyser to adjust the electric current consumed during desorption (thereby adjusting the heating of the adsorbent) in order to control the required concentration of anaesthetic agent, e.g. flurane, and to adjust the directions of the gas flows in the process.

The invention further relates to an apparatus for treating an anaesthetic gas, in which the process of the invention is used.

The invention is hereinafter described by means of drawings, wherein a flow diagram for a method of treating an exhaust gas comprising flurane and N₂O is schematically shown. The apparatus comprises an inlet (1) for exhaust from a patient, an inlet (5) for fresh anaesthetic gas, conduction lines (2, 4, 6, 8, 10 and 13), ad/desorption beds (3, 3', 3"), optional heating devices (7), a cooling device (9), a central control unit, an analyser (concentration measuring device; CM), a thermometer (TM) and a flow meter (FM), and outlets (11, 12) for waste anaesthetic gas and anaesthetic agent-enriched gas respectively.

In the present invention the flow of anaesthetic gas can be conducted via more than one adsorption bed to adsorb or desorb anaesthetic agents.

Figure 1 shows an embodiment wherein the exhaust gas from the patient is conducted through two adsorption beds. The exhaust gas enters via an inlet (1). The gas is conducted via valve 217, and valve 201 via line (2). Valve V205, V206, V211 and V213 are closed and the gas is conducted to a first adsorption bed (3). The gas flow is directed towards a second adsorption bed (3') via valve V203 (requiring valves V202, V204, V206 and V210 to be closed). This double adsorption route provides for more profound adsorption of fluranes. After passing the second adsorption bed (3'), the gas flow, devoid of fluranes, but still comprising N₂O, is then further conducted via line (4), via valve V207, towards a decomposition unit (requiring valve V208 and V209 to be closed). Of course, if the exhaust gas only comprises flurane as anaesthetic agent, the gas will not comprise N₂O, but will comprise usual components of exhaled air/breath. Fresh anaesthetic gas enters the system by an inlet (5). At a three-way crossing, valve V218 directs the gas flow via line (6) towards valve V216. However, also gas is directed directly towards the anaesthetic machine via line (13). The open valve V216 directs the fresh gas flow both towards the anaesthetic machine and to the desorption bed (3") containing anaesthetic agent. To this end valves V204, V209 and V212 are closed, whereas valve V214 is opened. By application of an electric current or preheating of the supplied anaesthetic gas, the anaesthetic agent desorbs from the adsorption bed (3"). In case a pre-heated gas is used for desorption purposes, a heating device (7) is present upstream of the desorption bed, to pre-heat the anaesthetic gas before contact with the adsorption bed. Heating may also be provided by heating the adsorption bed by a heating wire. After desorption of fluranes into the fresh gas flow, the anaesthetic gas flow enriched with anaesthetic agent, here fluranes, is conducted via open valve V215 and lines (10) and (8) towards the anaesthetic machine for further administering to a patient. Before entering the anaesthetic machine, a cooling device (9) is passed, e.g. a fin tube cooler, to cool the enriched anaesthetic gas mixture to a temperature suitable to administer the same to a patient. An analyser (CM) is present that measures at least the concentration of the anaesthetic agent in the gas to be inhaled by the patient. A Central Control Unit (CCU) is present that uses the signal from this analyser (CM) to adjust the electric current consumed during desorption (thereby adjusting the heating of the adsorbent) in order to control the required concentration of anaesthetic agent, here flurane, and to adjust the directions of the gas flows in the process. Such an analyser and associated CCU are commonly used and known to a man skilled in the art. Also a thermometer (TM) and a flow meter (FM) are present.

Figure 2 depicts the situation where the exhaust gas is conducted towards a single adsorption bed, whereas desorption occurs at two adsorption beds. The exhaust gas enters via an inlet (1). The gas is conducted via valve 217, line (2), and valve V206 to a first adsorption bed (3'). Valves V201 and V211 are closed. After passing the adsorption bed (3'), the gas flow, devoid of fluranes, but still comprising N₂O, is conducted via line (4), via valve V207 and outlet (11), towards a decomposition unit. Valves V208 and V209 need to be closed. Fresh anaesthetic gas enters the system via an inlet (5). Via valve V218 the gas flows via line (6) towards valve V216. However, gas is also directed directly towards the anaesthetic machine via line 13. The valve V216 directs the fresh gas flow both towards the anaesthetic machine and to two desorption beds (3 and 3"), via valves V204 and V214, whereas valve V209 is closed. After desorption of fluranes into the fresh gas flow, the enriched gas flow is conducted via valves V205 and V215 and line (10) towards the anaesthetic machine (valve V210 is closed). Cooling and regulation of the process occurs as describe above.

It is clear, that the mode of the beds (3, 3', 3") can be changed from adsorption bed to desorption bed and vice versa. The time of desorption is in general about 15 minutes, depending on the phase of the surgery process, the amount of the fluranes conducted through the beds, the amount of adsorbent, etc.

When the desorption bed(s) become(s) depleted of anaesthetic agent, the desorption bed(s) can be replaced by (a) fresh desorption beds. According to the apparatus of the present invention, this may be done in a so called merry-go-round system. Basically, the beds may be present in a unit, and by a controlled opening and closure of the appropriate valves the gas flows are redirected resulting in the beds to functionally replace each other.

The apparatus according to the present invention is a virtually closed system. The anaesthetic agent, here fluran, is kept in its adsorbed form until it is to be supplied to the patient.

The invention therefore provides for the possibility that the set of adsorption beds can be changed in the unit, depending on the anaesthetic agent used. Comparably, state of the art anaesthesia require that the bottle containing the liquid anaesthetic agent is replaced in anaesthetic machines to enable the use of different fluranes. The bottle containing the anaesthetic agent, e.g. flurane, may remain present in an apparatus of the present invention, to serve as a backup, or just for replenishment of the system due to the operational losses (ca. 1%).

As indicated above, the adsorption beds with the unit containing them can be removed together with the bottle mentioned above. In this way the saturated adsorption beds can function as a storage of anaesthetic agent, to be used in an other anaesthetic process with an other patient. No condensation and subsequent collection of fluranes is required, since the anaesthetic agent is stored on the ad/desorption beds. In case desorbed fluranes are collected in a container, for example a bottle, this bottle may be comprised in the unit containing the adsorption beds, and may be removed as part of the unit as well.

An anaesthetic machine administers the anaesthetic gas mixture to the patient. The anaesthethic machine may be provided with an anaesthethic agent vaporizer, preferably for the vaporation of fluranes, from the bottle mentioned above, to compensate for said potential minimal loss (ca 1%) of anesthethic agent (fluranes) during the process.

## Claims

1. A process for the treatment of an exhaled anaesthetic gas comprising at least one anaesthetic agent, said process comprising the steps of:
- in a first step contacting said anaesthetic gas with an adsorbent to adsorb said anaesthetic agent onto said adsorbent,
- in a second step desorbing said anaesthetic agent from said adsorbent, and collecting said desorbed anaesthetic agent in a form to reuse the same.

2. A process according to claim 1, in which said exhaled gas furthermore comprises at least another anaesthetic agent, for example N₂O, which is not adsorbed to the adsorbent and is subsequently treated.

3. A process according to claim 1, wherein the second step comprises leading the desorbed anaesthetic agent away from the adsorbent using a carrier gas, for example air, an N₂/O₂ gas mixture, an anaesthetic agent comprising gas/02 mixture or the like and administering the anaesthetic agent comprising mixture towards a patient.

4. A process for the treatment of an exhaled anaesthetic gas comprising at least two anaesthetic agents, comprising:
- in a first step contacting said anaesthetic gas with an adsorbent to adsorb at least one anaesthetic agent onto said adsorbent and wherein at least one other anaesthetic agent is not adsorbed,
- in a second step a further treatment of said at least one other anaesthetic agent, for example catalytic decomposition.

5. A process according to claims 1-4, in which said anaesthetic gas that is supplied to the adsorbent comprises a flurane.

6. A process according to claim 1-5, in which the adsorbent comprises zeolite or carbon, preferably activated carbon, more preferably mainly consists of activated carbon.

7. A process according to claims 1-6, in which the desorption of anaesthetic agent is effected by heating the adsorbent.

8. A process according to claim 7, in which the adsorbent is heated using an electric current.

9. A process according to claim 7 or 8, in which the adsorbent is heated by contacting the same with a gas, such that the temperature of the adsorbent is at or above the desorption temperature of said anaesthetic agent.

10. A process according to claims 1-9, in which the required concentration of anaesthetic agent is controlled by an analyser.

11. Apparatus for treating an anaesthetic gas for performing the process according to the claims 1-7, comprising:
an inlet (1) for exhaust gas from a patient, and an inlet (5) for fresh anaesthetic gas, conduction lines (2, 4, 6, 8, 10, 13), ad/desorption beds (3, 3',3"), optional heating devices (7), a cooling device (9), an analyser, a control unit and an outlet (11, 12),
wherein the exhaust gas from a patient is introduced into the apparatus via an inlet (1), conducted towards at least one adsorption bed (3, 3' or 3"), to adsorb an anaesthetic agent, preferably flurane, wherein the remaining anaesthetic gas, devoid of anaesthetic agent, is conducted via a line (4) and an outlet (11) to a decomposition unit,
and wherein the apparatus further comprises an inlet (5) for fresh anaesthetic carrier gas that is conducted via a line (6) towards at least one adsorption bed (3, 3' or 3"), that is not used for adsorption purposes,
**characterised in that** at the latter adsorption bed(s) the anaesthetic agent is desorbed by applying an electric current or by preheating the anaesthetic gas by a heating device (7), after which the anaesthetic gas, enriched with the anaesthetic agent is conducted via a line (8, 10) and an outlet (12) towards an anaesthetic machine and cooled by a cooling device (9) before entering the anaesthetic machine.

12. Apparatus according to claim 11, comprising means for replacing a regenerated adsorbent by an adsorbent containing anaesthetic agent.

13. Apparatus according to claim 12, in which said means is a removable unit comprising the ad/desorption beds, in which the function of adsorption and of desorption is alternated by controlled direction of the gas flow, using controllable valves.
